# EUROPEAN PATENT SPECIFICATION

(11) **EP 4 385 983 B1**
(45) Date of publication and mention of the grant of the patent: **08.04.2026**
(21) Application number: 22855060.4
(22) Date of filing: 09.06.2022
(51) Int. Cl.: C07D 401/04, C07D 405/14, A61P 35/00, A61K 31/4439

(54) **N-[2-(2,6-DIOXO-3-PIPERIDINYL)-2,3-DIHYDRO-1-OXO-1H-ISOINDOL-4-YL]-CARBAMIC ACID (5-METHYL-2-OXO-1,3-DIOXOL-4-YL)METHYL ESTER AND ITS PREPARATION FROM LENALIDOMIDE AND 4-(HYDROXYMETHYL)-5-METHYL-1,3-DIOXOL-2-ONE**
N-[2-(2,6-DIOXO-3-PIPERIDINYL)-2,3-DIHYDRO-1-OXO-1H-ISOINDOL-4-YL]-CARBAMINSÄURE (5-METHYL-2-OXO-1,3-DIOXOL-4-YL)METHYLESTER UND SEINE HERSTELLUNG AUS LENALIDOMID UND 4-(HYDROXYMETHYL)-5-METHYL-1,3-DIOXOL-2-ON
ESTER MÉTHYLIQUE DE L'ACIDE N-[2-(2,6-DIOXO-3-PIPÉRIDINYL)-2,3-DIHYDRO-1-OXO-1H-ISOINDOL-4-YL]-CARBAMIQUE (5-MÉTHYL-2-OXO-1,3-DIOXOL-4-YL) ET SA PRÉPARATION À PARTIR DE LÉNALIDOMIDE ET DE 4-(HYDROXYMÉTHYL)-5-MÉTHYL-1,3-DIOXOL-2-ONE

(30) Priority: 09.08.2021 CN 202110907061
(43) Date of publication of application: 19.06.2024
(73) Proprietor: Shanghai Maius Pharmaceutical Co. Ltd., Shanghai 201210 (CN)
(72) Inventor: SHI, Mingfeng, Shanghai 201210 (CN); GUO, Yekun, Shanghai 201210 (CN); HU, Xiang, Shanghai 201210 (CN); HUANG, Dujian, Shanghai 201210 (CN)
(74) Representative: Plasseraud IP
(86) International application number: PCT/CN2022/097865
(87) International publication number: WO 2023/016075

(56) References cited:
- WO-A1-2019/036839
- CN-A- 101 580 501
- CN-A- 103 396 397
- CN-A- 103 396 397
- CN-A- 106 456 795

## Description

### TECHNICAL FIELD

The present invention pertains to the field of medicine and, in particular, to a dihydro-2H-isoindole ester compound.

### BACKGROUND

Multiple myeloma (MM) is a poorly prognostic malignant tumor often found in elders. MM is characterized by significantly impaired hematopoiesis in bone marrow, or even hematopoietic failure, induced by tremendous proliferation of monoclonal plasma cells in bone marrow, which eventually leads to multifocal bone destruction.

At present, MM treatment relies mainly on chemotherapy. According to The Guidelines for the Diagnosis and Management of Multiple Myeloma in China(2020 revision), most currently available induction therapies adopt a three-drug regimen featuring the use of a proteasome inhibitor in combination with an immunomodulator and dexamethasone. Commonly used such regimens include bortezomib/thalidomide/dexamethasone (BTD), lenalidomide /bortezomib /dexamethasone (RVD) and thalidomide/cyclophosphamide/dexamethasone (TCD).

Thalidomide, lenalidomide and pomalidomide are immunomodulators commonly used in the clinical practice and each have a skeletal structure consisting of a dihydro-2H-isoindole ring linked to a piperidinedione ring:

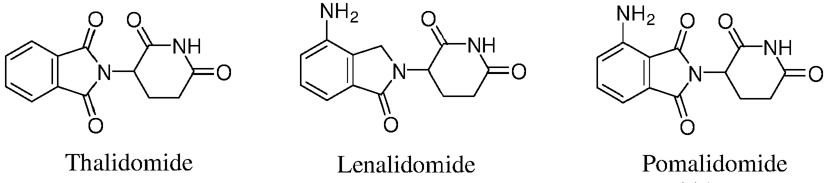

Known effects of these immunomodulators include anti-tumorous properties, antiangiogenic properties, erythropoietic stimulation and immune regulation. It is considered that they can attack the micro-environment of plasma cells in a multi-targeted manner, causing apoptosis of the cells. They can provide both immune regulation and angiogenic inhibition.

In-depth research on the three "-domide" drugs is continuously widening their scope of indications, gradually making them pan-cancer drugs for the treatment of various cancers.

During use, hematologic toxicity (decreases in neutrophils and platelets) and venous thrombosis (deep vein thrombosis and pulmonary embolism) are common adverse reactions to the three drugs. In particular, decreases in neutrophils and platelets have been recognized as the major dose-limiting toxicity.

For example, a clinical study showed that patients who received lenalidomide at 50 mg per day all showed Grade 3 myelosuppression on the 28^{th} day of treatment and exhibited good tolerance when the dose was lowered to 25 mg per day. Therefore, the latter dose was taken as the maximum tolerated dose (MTD). Studies have shown that severe myelosuppression (Grade 3 or higher) is dose-dependent, and it is suggested that the dose be reduced once any toxic or side effect such as myelosuppression manifested, for example, as a decrease in neutrophils or platelets is observed.

Clinical pharmacokinetic studies show that the "-domide" drugs are absorbed and metabolized rapidly and feature significant plasma drug concentration variation. High peak plasma drug concentrations have been identified as a dose-limiting factor.

CN103396397A relates to lenalidomide derivatives and its preparation method. WO2019/036839A relates to a pomamide amine derivative.

### SUMMARY

In view of the above described disadvantages and drawbacks in practical use of the "-domide" drugs, the inventors attempted a series of structural modifications of them, studied pharmacokinetics of the modified compounds and successfully identified a class of dihydro-*2H*-isoindole ester compounds.

Accordingly, there is provided a dihydro-*2H*-isoindole ester compound of general structural formula (I): 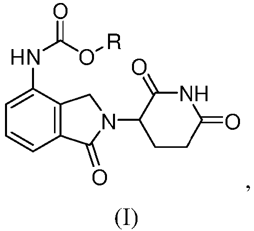 where R is selected from -CH(CH₃)₂, -(CH₂)₂CH₃, -(CH₂)₃CH₃, -CH₂CH(CH₃)₂, - (CH₂)₅CH₃, 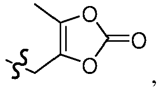 -(CH₂)₇CH₃, -(CH₂)₁₀CH₃, -(CH₂)₁₇CH₃, -CH₂CH(C₂H₅)₂, - CH(C₂H₅)₂, -CH(CH(CH₃)₂)₂ and -CH₂Ph.

In a first aspect, the present invention is disclosed in the attached set of claims.

There are provided methods of preparing the dihydro-*2H*-isoindole ester compound.

In a first method, commercially available 3-(4'-amino-1-oxo-1,3-dihydro-*2H*-isoindol-2-yl)piperidine-2,6-dione is used as a starting material, and a target compound is obtained from its reaction with a corresponding chloroformate, according to: 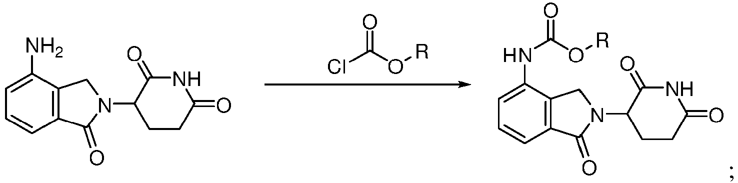 or
in a second method, the same starting material is first reacted with phenyl chloroformate (or *p*-nitrophenyl chloroformate) to derive its phenyl (or *p*-nitrophenyl) ester, which is then subjected to transesterification with a corresponding alcohol, giving a target compound, according to: 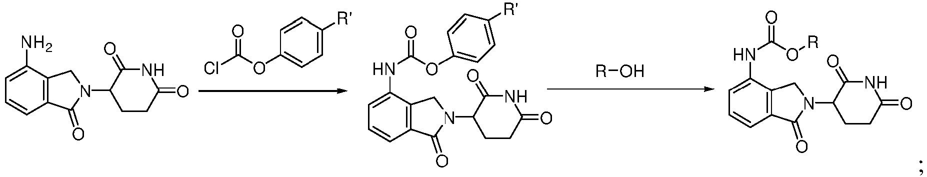
where R is selected from -CH(CH₃)₂, -(CH₂)₂CH₃, -(CH₂)₃CH₃, -CH₂CH(CH₃)₂, - (CH₂)₅CH₃, 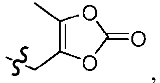 -(CH₂)₇CH₃, -(CH₂)₁₀CH₃, -(CH₂)₁₇CH₃, -CH₂CH(C₂H₅)₂, - CH(C₂H₅)₂, -CH(CH(CH₃)₂)₂ and -CH₂Ph; and
R' is H or nitro.

In a second aspect of the present invention, the methods are as defined in the attached set of claims.

There is provided use of the dihydro-2*H*-isoindole ester compound for preparation of a drug for treating multiple myeloma.

In a third aspect of the present invention, there is provided a dihydro-2*H*-isoindole ester compound for use in a method of treatment of multiple myeloma.

The present invention has the following benefits:
1. The dihydro-2*H*-isoindole ester compound of the present invention has better permeability than the existing drugs. After being orally administered, it is absorbed in a steady and smooth manner and hydrolyzed into an active metabolite, which exerts its therapeutic effect in a sustained manner. Due to the distinct absorption and metabolism behavior, the compound of the present invention possesses pharmacokinetic properties that can better address clinical needs, when compared to the existing drugs.
2. The active metabolite of the dihydro-2*H*-isoindole ester compound of the present invention has an AUC value equivalent to about 40-60% of that of the existing drugs administered at the same molar amount. However, it is advantageous in a time to peak concentration up to 7 h and an average retention time as long as 4-9 h, both better than those of the existing drugs, which are 0.7 h and 3 h, respectively.

The active metabolite of Compound 1 has an AUC value equivalent to 45% of that of the existing drugs administered at the same molar amount, a time to peak concentration of 0.8 h and an average retention time of 4.5 h. The active metabolite of Compound 3 has an AUC value equivalent to 40% of that of the existing drugs administered at the same molar amount, a time to peak concentration of 5 h and an average retention time of 5.6 h. The active metabolite of Compound 6 has an AUC value equivalent to 61% of that of the existing drugs administered at the same molar amount, a time to peak concentration of 7 h and an average retention time of 8.6 h.

Among the AUC value (equivalent to 61% of that of the existing drugs administered at the same molar amount), the time to peak concentration (7 h) and the average retention time (8.6 h) of the active metabolite of Compound 6, both the time to peak concentration and the average retention time are much better than those of the existing drugs.

3. The dihydro-2*H*-isoindole ester compound of the present invention can provide a steadier and smoother plasma drug concentration profile and can exert a longer-lasting therapeutic effect.

### BRIEF DESCRIPTION OF THE DRAWINGS

Fig. 1 shows a Plasma drug concentration-time curve of the reference drug after it is oral gavage administered to rats.
Fig. 2 shows a Plasma drug concentration-time curve of the active metabolite of Compound 1 after it is oral gavage administered to rats.
Fig. 3 shows a Plasma drug concentration-time curve of the active metabolite of Compound 3 after it is oral gavage administered to rats.
Fig. 4 shows a Plasma drug concentration-time curve of the active metabolite of Compound 6 after it is oral gavage administered to rats.

### DETAILED DESCRIPTION

The present invention will be described clearly and fully below with reference to specific embodiments thereof and to the accompanying drawings.

The following are the definitions of some terms as used in the context of the present invention.

The term "plasma drug concentration" refers to the total concentration of a drug in the blood plasma after it is absorbed in the form of being either bound to plasma protein or free in the plasma. Sometimes, this term refers to the concentration of a drug in the whole blood. The potency of a drug is proportional to its plasma drug concentration, and its in vivo concentration varies over time.

The term "time to peak concentration" refers to the time that it takes for a drug to reach its peak plasma drug concentration after its administration. At the end of this time, the drug is present at a maximum plasma drug concentration. The time to peak concentration of a drug can be used to analytically determine a suitable time for the drug to be taken.

The term "AUC" stands for the area under the curve of the plasma drug concentration of a drug as a function of time. In modern pharmacokinetic studies, AUC is an important parameter for assessing the in vivo properties of a drug. This area represents the total amount of absorption of a drug within a certain time after it is administered for a single time and can be used to calculate the drug's bioavailability.

The term "terminal elimination half-life" refers to the time that it takes for the plasma drug concentration of a drug that has reached a distribution equilibrium to drop by 50%.

In the context of the present invention, some compounds of general structural formula (I) that it is directed to, as well as the respective R groups therein, are numbered as shown in Table 1 below. Compounds 1-5 and 7-10 are provided as reference compounds for comparative purposes.

**Table 1**

| Compound No. | R Group |
|---|---|
| 1 | -CH(CH₃)₂ |
| 2 | - (CH₂)₂CH₃ |
| 3 | - (CH₂)₃CH₃ |
| 4 | -CH₂CH(CH₃)₂ |
| 5 | -(CH₂)₅CH₃ |
| 6 | 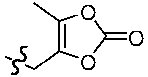 |
| 7 | -(CH₂)₇CH₃ |
| 8 | -(CH₂)₁₀CH₃ |
| 9 | -(CH₂)₁₇CH₃ |
| 10 | -CH₂CH(C₂H₅)₂ |
| 11 | -CH(C₂H₅)₂ |
| 12 | -CH(CH(CH₃)₂)₂ |
| 13 | -CH₂Ph |

### Example 1: Preparation of Compound 1

In a 250-mL three-neck flask, 12 g of 3-(4'-amino-1-oxo-1,3-dihydro-2*H*-isoindol-2-yl)piperidine-2,6-dione and then 100 g of tetrahydrofuran were added, followed by heating to reflux. Subsequently, 5.7 g of isopropyl chloroformate was added, and the reaction was run for 4 h under reflux. After that, 3.6 g of isopropyl chloroformate was supplemented, and the reaction was further run for another 4 h. Again, 2.7 g of isopropyl chloroformate was supplemented, and the reaction was further run for yet another 4 h. The reaction was stopped at the point when there was almost no starting material left according to TLC monitoring.

After the reaction was complete, 80 g of tetrahydrofuran was distilled out, and 50 g of ethanol was added, followed by agitation at 70 °C. After the temperature cooled down to 20-25 °C, filtration was performed, and the filter cake was collected and again agitated in 50 g of ethanol at 70 °C. After the temperature cooled down to 20-25 °C, filtration was performed, and the filter cake was collected and dried to afford the product. 12.0 g of the product was obtained at a yield of 75%.

¹H NMR (300 MHz, DMSO-d₆) δ 11.01 (s, 1H), 9.45 (s, 1H), 7.76 (dt, *J =* 7.4, 3.7 Hz, 1H), 7.54 - 7.42 (m, 2H), 5.13 (dd, *J* = 13.2, 5.1 Hz, 1H), 4.91 (hept, *J* = 6.3 Hz, 1H), 4.53 - 4.26 (m, 2H), 2.92 (ddd, *J* = 17.2, 13.5, 5.4 Hz, 1H), 2.69 - 2.55 (m, 1H), 2.36 (qd, *J* = 13.2, 4.4 Hz, 1H), 2.03 (ddd, *J* = 9.6, 5.1, 2.4 Hz, 1H), 1.27 (d, *J =* 6.2 Hz, 6H).¹³C NMR (75 MHz, DMSO-d₆) δ 173.34, 171.48, 168.30, 153.88, 134.49, 133.52, 133.22, 129.16, 124.77, 118.81, 68.47, 52.07, 46.78, 31.69, 23.06, 22.40.

As detected by mass spectrometry, [M+H]⁺=346.1, in consistence with the molecular structural formula.

### Example 2: Preparation of Compound 2

The same preparation steps as in Example 1 were performed, except that isopropyl chloroformate was replaced with propyl chloroformate. 13.4 g of the target product was obtained at a yield of 83.6%.

¹H NMR (300 MHz, DMSO-d₆) δ 11.01 (s, 1H), 9.51 (s, 1H), 7.76 (dt, *J =* 7.4, 3.7 Hz, 1H), 7.56 - 7.43 (m, 2H), 5.13 (dd, *J* = 13.2, 5.1 Hz, 1H), 4.54 - 4.28 (m, 2H), 4.06 (t, *J* = 6.7 Hz, 2H), 2.92 (ddd, *J* = 18.1, 13.4, 5.3 Hz, 1H), 2.74 - 2.55 (m, 1H), 2.36 (qd, *J* = 13.3, 4.5 Hz, 1H), 2.15 - 1.91 (m, 1H), 1.65 (q, *J =* 7.1 Hz, 2H). ¹³C NMR (75 MHz, DMSO-d₆) δ 173.33, 171.48, 168.28, 154.39, 134.42, 133.56, 133.23, 129.20, 124.79, 118.89, 66.57, 52.06, 46.75, 31.68, 23.07, 22.34, 10.72.

As detected by mass spectrometry, [M+H]⁺=346.1, in consistence with the molecular structural formula.

### Example 3: Preparation of Compound 3

The same preparation steps as in Example 1 were performed, except that isopropyl chloroformate was replaced with butyl chloroformate. 12.0 g of the target product was obtained at a yield of 72.1%.

¹H NMR (300 MHz, DMSO-d₆) δ 11.01 (s, 1H), 9.50 (s, 1H), 7.76 (dt, *J =* 7.3, 3.6 Hz, 1H), 7.49 (d, *J =* 3.0 Hz, 2H), 5.13 (dd, *J* = 13.2, 5.1 Hz, 1H), 4.56 - 4.23 (m, 2H), 4.11 (t, *J =* 6.6 Hz, 2H), 2.92 (ddd, *J* = 18.4, 13.6, 5.4 Hz, 1H), 2.61 (d, *J =* 16.9 Hz, 1H), 2.35 (qd, *J* = 13.1, 4.3 Hz, 1H), 2.03 (dtd, *J* = 10.2, 7.9, 6.5, 3.5 Hz, 1H), 1.62 (tt, *J =* 8.4, 6.4 Hz, 2H), 1.49 - 1.27 (m, 2H), 0.92 (t, *J =* 7.3 Hz, 3H). ¹³C NMR (75 MHz, DMSO-d₆) δ 173.33, 171.48, 168.29, 154.39, 134.44, 133.52, 133.23, 129.18, 124.74, 118.87, 64.77, 52.07, 46.76, 31.69, 31.02, 23.09, 19.07, 14.04.

As detected by mass spectrometry, [M+H]⁺=360.1, in consistence with the molecular structural formula.

### Example 4: Preparation of Compound 4

The same preparation steps as in Example 1 were performed, except that 15 g of the starting material was used and isopropyl chloroformate was replaced with a corresponding amount of isobutyl chloroformate. 14.9 g of the target product was obtained at a yield of 71.6%.

¹H NMR (300 MHz, DMSO-d₆) δ 11.01 (s, 1H), 9.51 (s, 1H), 7.77 (dt, *J =* 7.4, 3.7 Hz, 1H), 7.56 - 7.42 (m, 2H), 5.13 (dd, *J* = 13.2, 5.1 Hz, 1H), 4.57 - 4.25 (m, 2H), 3.90 (d, *J* = 6.7 Hz, 2H), 2.92 (ddd, *J* = 18.1, 13.4, 5.4 Hz, 1H), 2.61 (d, *J =* 17.2 Hz, 1H), 2.36 (qd, *J* = 13.2, 4.5 Hz, 1H), 2.13 - 1.96 (m, 1H), 1.94 (dp, *J =* 13.4, 6.8 Hz, 1H), 0.94 (d, *J =* 6.7 Hz, 6H). ¹³C NMR (75 MHz, DMSO-d₆) δ 173.33, 171.49, 168.28, 154.42, 134.42, 133.56, 133.23, 129.20, 124.80, 118.90, 70.96, 52.05, 46.73, 31.69, 28.03, 23.08, 19.36.

As detected by mass spectrometry, [M+H]⁺=360.1, in consistence with the molecular structural formula.

### Example 5: Preparation of Compound 5

The same preparation steps as in Example 1 were performed, except that isopropyl chloroformate was replaced with *n*-hexyl chloroformate. 11.0 g of the target product was obtained at a yield of 62.5%.

¹H NMR (300 MHz, DMSO-d₆) δ 11.01 (s, 1H), 9.50 (s, 1H), 7.76 (dt, *J =* 7.4, 3.7 Hz, 1H), 7.57 - 7.35 (m, 2H), 5.13 (dd, *J* = 13.2, 5.1 Hz, 1H), 4.55 - 4.25 (m, 2H), 4.10 (t, *J =* 6.6 Hz, 2H), 2.92 (ddd, *J* = 17.8, 13.4, 5.3 Hz, 1H), 2.61 (d, *J =* 17.6 Hz, 1H), 2.35 (qd, *J* = 13.2, 4.4 Hz, 1H), 2.03 (dtd, *J* = 12.0, 5.1, 4.6, 1.9 Hz, 1H), 1.74 - 1.52 (m, 2H), 1.46 - 1.20 (m, 6H), 0.88 (t, *J =* 6.8 Hz, 3H). ¹³C NMR (75 MHz, DMSO-d₆) δ 173.31, 171.47, 168.28, 154.38, 134.44, 133.52, 133.23, 129.18, 124.75, 118.87, 65.07, 52.06, 46.74, 31.69, 31.39, 28.95, 25.51, 23.09, 22.51, 14.34.

As detected by mass spectrometry, [M+H]⁺=388.2, in consistence with the molecular structural formula.

### Example 6: Preparation of Compound 6

In a 1-L single-neck flask, 17 g of 3-(4'-amino-1-oxo-1,3-dihydro-2*H*-isoindol-2-yl)piperidine-2,6-dione, 19.8 g of *p*-nitrophenyl chloroformate and 250 mL of tetrahydrofuran were added, followed by stirring for 3 h under heating at 65 °C. When it was detected by TLC monitoring that the starting material had been completely consumed, rotation-evaporation was performed, and 200 mL of ethyl acetate was added, followed by agitation therein. From filtration, 24 g of a white solid was obtained.

In a 500-mL three-neck flask, 19 g of the white solid obtained from the above step, 9.7 g of 4-(hydroxymethyl)-5-methyl-[1,3]dioxol-2-one, 7.6 g of triethylamine and 250 mL of DMF were added and stirred at 25 °C for 24 h. When it was detected by TLC monitoring that the starting material had been completely consumed, the liquid reaction was poured into ice water and extracted with 1 L of ethyl acetate, followed by drying on anhydrous sodium sulfate and rotation-evaporation. A liquid fraction obtained from column chromatography (petroleum ether/ethyl acetate=1:1-0:1) was concentrated to give about 7 g of an oil, to which *n*-heptane (200mL) was added, followed by agitation. From filtration, 4.4 g of the target compound was obtained as a white solid with purity of 93.6%.

¹H NMR (300 MHz, DMSO-*d*₆) δ 11.01 (s, 1H), 9.78 (s, 1H), 7.77 (dt, *J* = 7.7, 3.8 Hz, 1H), 7.59 - 7.45 (m, 2H), 5.13 (dd, *J* = 13.2, 5.1 Hz, 1H), 5.05 (s, 2H), 4.56 - 4.24 (m, 2H), 2.92 (ddd, *J* = 18.0, 13.4, 5.3 Hz, 1H), 2.69 - 2.56 (m, 1H), 2.34 (qd, *J* = 13.2, 4.4 Hz, 1H), 2.20 (s, 3H), 2.10 - 1.95 (m, 1H). ¹³C NMR (76 MHz, DMSO-d₆) δ 173.33, 171.47, 168.20, 153.57, 152.36, 140.61, 134.16, 133.94, 133.77, 133.31, 129.31, 124.95, 119.30, 54.77, 52.08, 46.68, 31.68, 23.07, 9.32.

As detected by mass spectrometry, [M+H]⁺=416.1, in consistence with the molecular structural formula.

### Example 7: Preparation of Compound 7

The same preparation steps as in Example 1 were performed, except that isopropyl chloroformate was replaced with crude *n*-octyl chloroformate prepared from 5 eq. of *n-*octanol, 7.5 eq. of triethylamine and 2.5 eq. of triphosgene. The reaction was subject to column chromatography, giving 4.20 g of the target compound at a yield of 52.4%.

¹H NMR (400 MHz, DMSO-d₆) δ 11.01 (s, 1H), 9.51 (s, 1H), 7.76 (dt, *J =* 7.1, 3.5 Hz, 1H), 7.62 - 7.36 (m, 2H), 5.12 (dd, *J* = 13.2, 5.1 Hz, 1H), 4.40 (dd, *J =* 36.2, 17.6 Hz, 2H), 4.19 - 4.01 (m, 2H), 3.00 - 2.84 (m, 1H), 2.66 (dd, *J* = 36.3, 20.1 Hz, 1H), 2.35 (qd, *J =* 13.2, 4.4 Hz, 1H), 2.07 - 1.94 (m, 1H), 1.75 - 1.41 (m, 2H), 1.31 - 1.26 (m, 10H), 0.86 (t, *J* = 6.8 Hz, 3H).

As detected by mass spectrometry, [M+H]⁺=416.23, in consistence with the molecular structural formula.

### Example 8: Preparation of Compound 8

The same preparation steps as in Example 1 were performed, except that isopropyl chloroformate was replaced with crude *n*-undecyl chloroformate prepared from 5 eq. of *n-*undecanol, 7.5 eq. of triethylamine and 2.5 eq. of triphosgene. The reaction was subject to column chromatography, giving 5.6 g of the target compound at a yield of 63.2%.

¹H NMR (400 MHz, DMSO-d₆) δ 11.01 (s, 1H), 9.49 (s, 1H), 7.76 (dt, *J =* 7.1, 3.5 Hz, 1H), 7.58 - 7.39 (m, 2H), 5.12 (dd, *J* = 13.3, 5.1 Hz, 1H), 4.39 (q, *J =* 17.6 Hz, 2H), 4.08 (t, *J = 6.6* Hz, 2H), 3.00 - 2.83 (m, 1H), 2.63 (t, *J =* 16.4 Hz, 1H), 2.34 (qd, *J* = 13.2, 4.4 Hz, 1H), 2.02 (ddd, *J* = 13.2, 6.5, 4.5 Hz, 1H), 1.77 - 1.41 (m, 2H), 1.40 - 1.15 (m, 16H), 0.85 (t, *J* = 6.8 Hz, 3H).

As detected by mass spectrometry, [M+H]⁺=458.29, in consistence with the molecular structural formula.

### Example 9: Preparation of Compound 9

The same preparation steps as in Example 1 were performed, except that isopropyl chloroformate was replaced with crude *n*-octadecyl chloroformate prepared from 5 eq. of *n*-octadecanol, 7.5 eq. of triethylamine and 2.5 eq. of triphosgene. The reaction was subject to column chromatography, giving 6.4 g of the target compound at a yield of 60.1%.

¹H NMR (400 MHz, DMSO-d₆) δ 11.00 (s, 1H), 9.49 (s, 1H), 7.76 (dd, *J =* 5.9, 2.3 Hz, 1H), 7.48 (dd, *J* = 8.6, 5.0 Hz, 2H), 5.12 (dd, *J* = 13.2, 5.0 Hz, 1H), 4.39 (q, *J =* 17.6 Hz, 2H), 4.08 (t, *J =* 6.6 Hz, 2H), 3.02 - 2.81 (m, 1H), 2.60 (d, *J =* 17.0 Hz, 1H), 2.34 (qd, *J =* 13.2, 4.3 Hz, 1H), 1.99 (dd, *J* = 27.4, 21.9 Hz, 1H), 1.57 (dt, *J =* 69.7, 34.9 Hz, 2H), 1.41 - 0.95 (m, 30H), 0.85 (t, *J =* 6.6 Hz, 3H).

As detected by mass spectrometry, [M+H]⁺=556.39, in consistence with the molecular structural formula.

### Example 10: Preparation of Compound 10

The same preparation steps as in Example 1 were performed, except that isopropyl chloroformate was replaced with crude 2-ethylbutyl chloroformate prepared from 5 eq. of 2-ethylbutanol, 7.5 eq. of triethylamine and 2.5 eq. of triphosgene. The reaction was subject to column chromatography, giving 3.9 g of the target compound at a yield of 52.5%.

¹H NMR (400 MHz, DMSO-d₆) δ 11.01 (s, 1H), 9.47 (s, 1H), 7.75 (dt, *J =* 33.1, 16.5 Hz, 1H), 7.61 = 7.27 (m, 2H), 5.13 (dd, *J* = 13.2, 5.1 Hz, 1H), 4.40 (q, *J* = 17.6 Hz, 2H), 4.03 (d, *J* = 5.9 Hz, 2H), 3.00 - 2.83 (m, 1H), 2.61 (d, *J =* 16.9 Hz, 1H), 2.35 (qd, *J* = 13.2, 4.4 Hz, 1H), 2.10 - 1.94 (m, 1H), 1.58 - 1.47 (m, 1H), 1.45 - 1.28 (m, 4H), 0.88 (t, *J =* 7.4 Hz, 6H).

As detected by mass spectrometry, [M+H]⁺=388.19, in consistence with the molecular structural formula.

### Example 11: Pharmacokinetic Assays of Compounds

Instruments: ultra high performance liquid chromatography (Acquity UPLC, Waters) system; triple quadrupole mass spectrometer (Qtrap 5500, Sciex); high-speed refrigerated centrifuge (5430R, Eppendorf).

Chromatography Condition: Column: 2.1 × 50 mm (1.7 µm, BEH C18, Waters); Column Temperature: 45 °C; Mobile Phase: 0.1% aqueous solution of formic acid (A)/acetonitrile; Gradient: see the table below:

| Gradient Table | | | |
|---|---|---|---|
| Time | Flow | %A | %B |
| initial | 0.400 | 95.0 | 5.0 |
| 1.00 | 0.400 | 95.0 | 5.0 |
| 2.80 | 0.400 | 38.0 | 62.0 |
| 2.90 | 0.400 | 0.0 | 100.0 |
| 3.90 | 0.400 | 0.0 | 100.0 |
| 4.00 | 0.400 | 95.0 | 5.0 |

Mass Spectrometry Condition: Mode: positive ion multiple reaction monitoring (MRM); Curtain Gas: 45 psi; Gas 1: 45 psi; Gas 2: 45 psi; Ion Source Temperature: 500 °C; Ion Source Voltage: 5000 V. Precursor ions (Q1), product ions (Q3) and collision energy (CE) of some compounds are listed in the table below:

| | Q1 Mass (Da) | Q3 Mass (Da) | Time (msec) | ID | CE(volts) |
|---|---|---|---|---|---|
| 1 | 260.100 | 149.100 | 100.0 | Lenalidomide | 20.000 |
| 2 | 388.200 | 277.200 | 100.0 | Compound 5 | 30.000 |
| 3 | 416.100 | 193.100 | 100.0 | Compound 6 | 25.000 |
| 4 | 274.100 | 84.000 | 100.0 | IS | 20.000 |

Experimental Animals: male SD rats, 6 in each group, weighing 200-220 grams, fasting overnight before experimentation.

Preparation of Drugs: lenalidomide, as a reference drug, and the test compounds (Compounds 1-10) were accurately weighed and dispensed in 0.5% CMC-Na solutions, resulting in suspensions.

Drug Administration to and Blood Collection from Animals: the drugs were oral gavage administered to the rats, and 100-µl blood samples were taken before administration and 10 min, 20 min, 40 min, 1 h, 1.5 h, 2h, 3 h, 5 h, 8 h, 12 h, 21h, 24 h and 30h after administration. Each sample was placed in an ice bath, centrifuged for plasma collection, frozen and stored.

### Plasma drug concentration Data and Calculation of Pharmacokinetic Parameters:

Plasma drug concentration data was processed using MultiQuan 3.0 (Sciex), and pharmacokinetic parameters were calculated using DAS 2.0 software. Plasma drug concentrations were measured in ug/L.

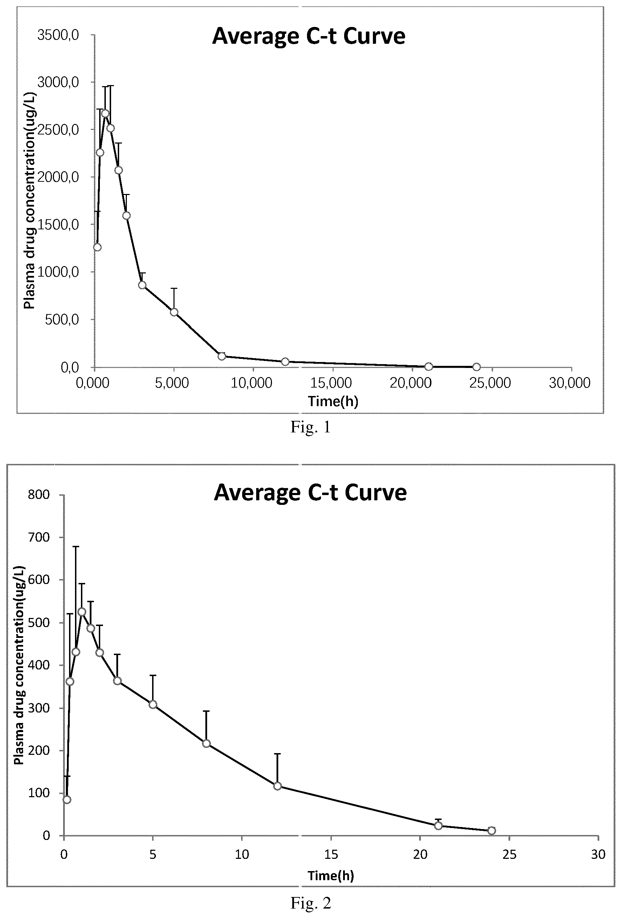

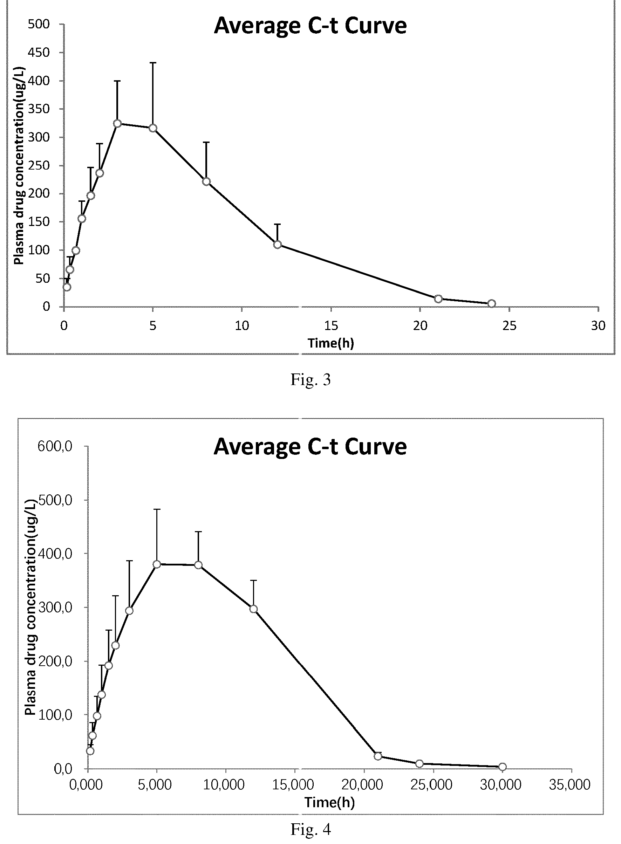

Figs. 1 to 4 show respective plasma drug concentration-time curves of the reference drug group and the test groups treated with Compounds 1, 3 and 6, and Table 2 shows the results of pharmacokinetic assays.

As can be seen from Figs. 1 to 4, and from the results of pharmacokinetic assays shown in Table 2, after being orally administered and absorbed, the compounds of the present invention can exert their intended therapeutic effects in the plasma. Active metabolites of compounds 1, 3, 6, etc. have an AUC value comparable to that of the conventional drugs administered at the same molar amount. However, the compounds of the present invention exhibit a lower plasma drug concentration peak, a prolonged time to peak concentration, a longer average retention time and pharmacokinetic properties that can better address clinical needs.

**Table 2 Results of Pharmacokinetic Assays**

| Parameter | Unit | reference drug | Compound 6 | Compound 1 | Compound 3 | Compound 5 |
|---|---|---|---|---|---|---|
| AUC(0-t) | ug/L*h | 8494.4±920 | 5217±754.8 | 3822±424.5 | 3398±304.2 | 1022±214.5 |
| AUC(0-∞) | ug/L*h | 8505.7±920.1 | 5229.3±755.1 | 3889.6±435.6 | 3413.1±314.3 | 1257±209.6 |
| AUMC(0-t) | | 25619.7±5124.9 | 44683.6±4956.6 | 32705.8±3125.3 | 28866.3:1:3051.1 | 8749±352.6 |
| AUMC(0-∞) | | 25989.2±5200.6 | 45199.7±4922.6 | 33484.2±3040.7 | 28901.2:1:3221.3 | 8954±336.4 |
| MRT(0-t) | h | 3±0.4 | 8.6±0.6 | 4.5±0.7 | 5.6±0.5 | 4±0.5 |
| MRT(0-∞) | h | 3±0.4 | 8.7±0.6 | 4.6±0.6 | 5.7±0.5 | 1.1±0.6 |
| VRT(0-t) | h^2 | 9.4±1.3 | 20±2.5 | 13±2.2 | 17±3.0 | 11±1.8 |
| VRT(0-∞) | h^2 | 10.3±1.6 | 21.8±2.8 | 14.3±2.1 | 17.8±2.8 | 11.8±2.1 |
| t1/2z | h | 2.8±0.2 | 2.9±0.4 | 2.8±0.3 | 2.9±0.3 | 3.0±0.3 |
| Tmax | h | 0.7±0.3 | 7±1.5 | 0.8±0.4 | 5±1.5 | 4±0.5 |
| CLz/F | L/h/kg | 3.6±0.4 | 5.9±1 | 4.3±1 | 4.9±0.9 | 4.2±0.9 |
| Vz/F | L/kg | 14.3±2.4 | 24.2±5.1 | 16.4±3.9 | 18.5±4.8 | 16.9±1.8 |
| Cmax | ug/L | 2766.9±335.6 | 405.4±89.8 | 543.6±68.5 | 378.4±75.3 | 158±31.1 |

| Parameter | Unit | Compound 7 | Compound 8 | Compound 9 | Compound 10 |
|---|---|---|---|---|---|
| AUC(0-t) | ug/L*h | 503.2±42.6 | 489.4±50.3 | 402.5±51.6 | 1568±205.4 |
| AUC(0-∞) | ug/L*h | 584.3±50.3 | 421.5±55.6 | 456±48.0 | 1765±226.3 |
| AUMC(0-t) | | 3965±415.1 | 3596±401.3 | 3126±298.1 | 13691±587.2 |
| AUMC(0-∞) | | 4123±446.1 | 3689±411.6 | 3360±318.6 | 14002±589.6 |
| MRT(0-t) | h | 3.8±0.7 | 3.8±0.5 | 3.5±0.6 | 5.2±0.7 |
| MRT(0-∞) | h | 3.8±0.8 | 3.9±0.5 | 3.6±0.7 | 5.2±0.7 |
| VRT(0-t) | h^2 | 10±1.3 | 9.8±1.5 | 10±1.5 | 14±2.1 |
| VRT(0-∞) | h^2 | 10.5±1.3 | 9.9±1.6 | 10.2±1.5 | 14.2±2.2 |
| t1/2z | h | 3.1±0.2 | 2.9±0.3 | 3.0±0.4 | 3.1±0.5 |
| Tmax | h | 3.5±0.8 | 3.8±1.1 | 4.1±0.8 | 5±0.9 |
| CLz/F | L/h/kg | 4.1±1.1 | 3.8±0.9 | 4.2±1.0 | 3.9±0.8 |
| Vz/F | L/kg | 15.1±1.4 | 14.9±1.2 | 13.9±2.0 | 16.1±1.9 |
| Cmax | ug/L | 135±23.0 | 105.3±24.1 | 95.6±22.2 | 163.2±26.4 |

## Claims

1. A dihydro-2*H*-isoindole ester compound, **characterized in** being of the general structural formula: 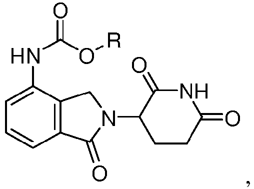 where R is

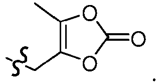

2. A method of preparing the dihydro-2*H*-isoindole ester compound according to claim 1, **characterized in** using 3-(4'-amino-1-oxo-1,3-dihydro-2*H*-isoindol-2-yl)piperidine-2,6-dione as a starting material and obtaining a target compound from its reaction with a corresponding chloroformate, wherein the reaction is represented by the following equation: 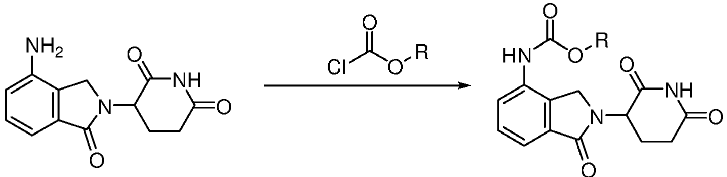 where R is

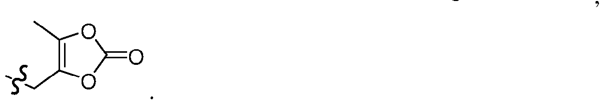

3. A method of preparing the dihydro-2*H*-isoindole ester compound according to claim 1, **characterized in** using 3-(4'-amino-1-oxo-1,3-dihydro-2*H*-isoindol-2-yl)piperidine-2,6-dione as a starting material and obtaining a target compound from transesterification of a phenyl (or *p*-nitrophenyl) ester compound, which is obtained from its reaction with phenyl chloroformate (or *p*-nitrophenyl chloroformate), with a corresponding alcohol, wherein the reactions are represented by the following equations: 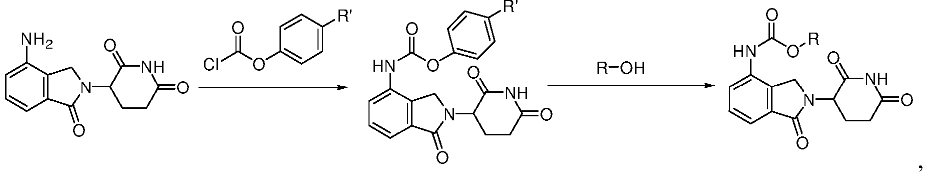
where R is 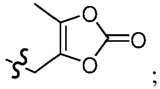 and
R' is H or nitro.

4. The dihydro-*2H*-isoindole ester compound according to claim 1 for use in a method of treatment of multiple myeloma.

## Patentansprüche

1. Dihydro-2*H*-isoindol-Ester-Verbindung, **gekennzeichnet durch** die allgemeine Strukturformel: 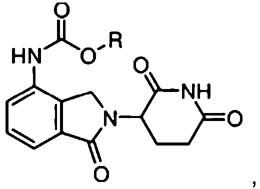 worin R 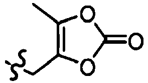 ist.

2. Verfahren zur Herstellung der Dihydro-2*H*-isoindol-Ester-Verbindung nach Anspruch 1, **gekennzeichnet durch** die Verwendung von 3-(4'-Amino-1-oxo-1,3-dihydro-2*H*-isoindol-2-yl)piperidin-2,6-dion als Ausgangsstoff und das Erhalten einer Zielverbindung durch dessen Reaktion mit einem entsprechenden Chlorformiat, wobei die Reaktion durch die folgende Gleichung dargestellt wird 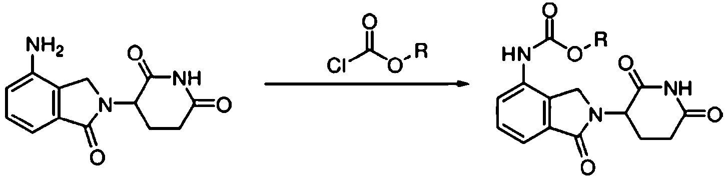 worin R 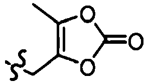 ist.

3. Verfahren zur Herstellung der Dihydro-2*H*-isoindol-Ester-Verbindung nach Anspruch 1, **gekennzeichnet durch** die Verwendung von 3-(4'-Amino-1-oxo-1,3-dihydro-2*H*-isoindol-2-yl)piperidin-2,6-dion als Ausgangsstoff und das Erhalten einer Zielverbindung durch Umesterung einer Phenyl- (oder *p*-Nitrophenyl-) Esterverbindung, die durch dessen Reaktion mit Phenylchlorformiat (oder *p-*Nitrophenylchlorformiat) erhalten wird, mit einem entsprechenden Alkohol, wobei die Reaktionen durch die folgenden Gleichungen dargestellt werden: 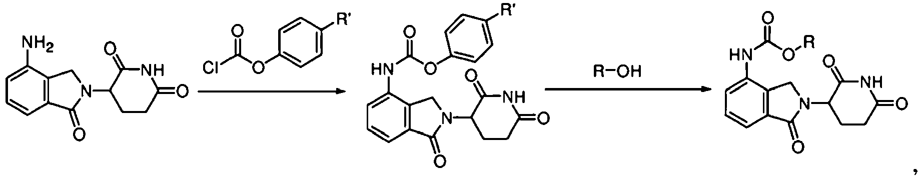
worin R 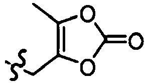 ist; und
R' H oder Nitro ist.

4. Dihydro-2*H*-isoindol-Ester-Verbindung nach Anspruch 1 zur Verwendung in einem Verfahren zur Behandlung von multiplem Myelom.

## Revendications

1. Composé ester dihydro-2*H*-isoindole, **caractérisé en ce qu'**il est de la formule développée générale : 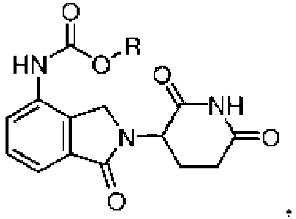 où R est

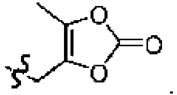

2. Procédé de préparation du composé ester dihydro-2*H*-isoindole selon la revendication 1, **caractérisé par** l'utilisation de 3-(4'-amino-1-oxo-1,3-dihydro-2*H-*isoindol-2-yl)pipéridine-2,6-dione comme matière de départ et l'obtention d'un composé cible à partir de sa réaction avec un chloroformiate correspondant, dans lequel la réaction est représentée par l'équation suivante : 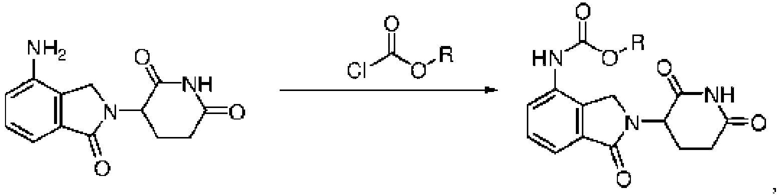 où R est

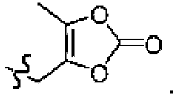

3. Procédé de préparation du composé ester dihydro-2*H*-isoindole selon la revendication 1, **caractérisé par** l'utilisation de 3-(4'-amino-1-oxo-1,3-dihydro-2*H-*isoindol-2-yl)pipéridine-2,6-dione comme matière de départ et l'obtention d'un composé cible par transestérification d'un composé ester phényl (ou *p*-nitrophényl), qui est obtenu par sa réaction avec le chloroformiate de phényle (ou *p*-nitrophényl chloroformiate), avec un alcool correspondant, dans lequel les réactions sont représentées par les équations suivantes : 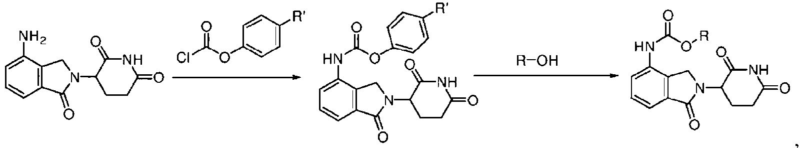
où R est 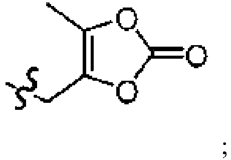 et
R' est H ou nitro.

4. Composé ester dihydro-2*H*-isoindole selon la revendication 1 pour une utilisation dans un procédé de traitement du myélome multiple.
